# EUROPEAN PATENT SPECIFICATION

(11) **EP 1 644 401 B1**
(45) Date of publication and mention of the grant of the patent: **09.03.2011**
(21) Application number: 04739972.0
(22) Date of filing: 17.06.2004
(51) Int. Cl.: C07K 1/04

(54) **IMPROVED PROCESS FOR SUPPORTED PHASE SYNTHESIS**
VERBESSERTES VERFAHREN FÜR FESTPHASENSYNTHESE
PROCEDE AMELIORE DE SYNTHESE DE PHASES SUPPORTEE

(30) Priority: 04.07.2003 EP 03015188
(43) Date of publication of application: 12.04.2006
(60) Divisional application: 10011937.9
(73) Proprietor: Lonza AG, 4052 Basel (CH)
(72) Inventor: COOL, Vincent, B-7850 Marcq (BE); FORNI, Luciano, B-7100 La Louvière (BE); MONNAIE, Didier, B-7110 Houdeng Aimeries (BE); RONVAUX, Alain, B-7900 Leuze-en-Hainaut (BE)
(74) Representative: Weber, Joachim
(86) International application number: PCT/EP2004/006512
(87) International publication number: WO 2005/005457

(56) References cited:
- CHEM FILES, GREEN CHEMISTRY12, [Online] vol. 1, no. 7, 2001, pages 1-18, XP002259798 Retrieved from the Internet: URL:http://www.sigmaaldrich.com/img/assets /6040/chemfiles_v1n7_greenchemistry_small. pdf> [retrieved on 2003-10-30]
- SPATOLA A F ET AL: "PHASE TRANSFER CATALYSIS IN SOLID PHASE PEPTIDE SYNTHESIS. ÖPREPARATION OF CYCLO(XXX-PRO-GLY-YYY-PRO-GLY) MODEL PEPTIDES AND THEIR CONFORMATIONAL ANALYSIS" INTERNATIONAL JOURNAL OF PEPTIDE AND PROTEIN RESEARCH, MUNKSGAARD, COPENHAGEN, DK, vol. 40, no. 3 / 4, 1 September 1992 (1992-09-01), pages 322-332, XP000311243 ISSN: 0367-8377
- CHAN WC, WHITE PD: "Fmoc solid phase peptide synthesis. A Practical Approach" 2000, OXFORD UNIVERSITY PRESS , XP009019468 Chapter 3
- CHEN S-T ET AL: "PHASE-TRANSFER REAGENTS AS CARBOXYL-TERMINAL PROTECTING GROUPS FACILE INCORPORATION OF FREE AMINO ACIDS OR PEPTIDES INTO PEPTIDE SEQUENCES" JOURNAL OF THE CHEMICAL SOCIETY CHEMICAL COMMUNICATIONS, no. 15, 1990, pages 1045-1047, XP009019280 ISSN: 0022-4936
- COSTE JACQUES ET AL: "Coupling N-methylated amino acids using PyBroP-1 and PyCloP halogenophosphonium salts: Mechanism and fields of application" JOURNAL OF ORGANIC CHEMISTRY, vol. 59, no. 9, 1994, pages 2437-2466, XP002259799 ISSN: 0022-3263
- VARANDA L M ET AL: "SOLID-PHASE PEPTIDE SYNTHESIS AT ELEVATED TEMPERATURES: A SEARCH FOR AN OPTIMIZED SYNTHESIS CONDITION OF UNSULFATED CHOLECYSTOKININ-12" JOURNAL OF PEPTIDE RESEARCH, MUNKSGAARD INTERNATIONAL PUBLISHERS, COPENHAGEN, DK, vol. 50, no. 2, 1 August 1997 (1997-08-01), pages 102-108, XP000659209 ISSN: 1397-002X

## Description

The present invention concerns an improved process for supported phase synthesis.

In solid phase synthesis (SPS), also called supported phase synthesis, an oligomer is assembled on a support by attaching sequentially appropriately protected monomers or other oligomers. This is done by sequential steps of coupling and deprotection, eventually separated by washing cycles.

The monomer is a building block of the molecule to be assembled on the support. For example, but not restricted to, the monomer is an appropriately protected amino acid, an appropriately protected nucleotide, an appropriately protected peptide nucleic acid, an appropriately protected saccharide or any closely modified structure of the same family.

The oligomer is a structure made by assembling at least 2 or more of the monomers from a same family, or a combination thereof. The definition of oligomer comprises bio-oligomers, which includes but is not restricted to, peptides, oligonucleotides (DNA, RNA), oligosaccharides, peptide nucleic acids (PNA) or a combination thereof. The oligomer may be linear or branched.

The support is a product to which the oligomer or first monomer is attached. This allows a thorough washing, to be performed when the oligomer attached to the support is in the solid state. Examples of supports are polystyrene beads, zeolites, controlled pore glass beads, cellulose, PEG resins, or polyamide, without being restricted thereto. Another example of a useful support is a polymer that may exist in a soluble state and can be precipitated by addition of an appropriate solvent in order to allow a thorough washing to take place.

The support on which the solid phase synthesis is performed is functionalized by a linker.

A preferred example of a support is a polystyrene - 1% divinylbenzene resin which is often used in solid phase peptide synthesis.

The term attaching or attachment means generally the formation of a covalent bond and is used for covalent bonds between any of the following: it may be the covalent bond between the first monomer and the support, wherein this bond is formed via a linker, or the bond formed between any of the monomers in the oligomer. The bond between the first monomer and the support, which is formed via a linker, and the bond between monomers in the oligomer, may make use of any functionality of the monomer, e.g. functional groups in its backbone or side chain. In solid phase peptide synthesis, the first amino acid may be attached to the support via benzylic type linkers. The linker is an appropriate molecular structure that is covalently attached to the support, and that will release the target oligomer under adequate chemical conditions. Benzylic type linkers such as benzyl ester or monoalkyloxybenzyl ester are often used, however, other linkers can also be considered. Benzylic type linkers release the target oligomer under acidolytic conditions.

Under thorough washing is understood any technical method that will eliminate the residual by-products or reagents from the previous step, e.g. coupling or deprotection reagents. A thorough washing may be performed in a batch mode or in a continuous mode process. Examples of a continuous mode process are the thorough washing of the support with a solvent in a column or the elimination of by-products or reagents through physical means like centrifugation.

In solid phase peptide synthesis (SPPS) the peptide is assembled on a polystyrene bead as support. Construction of a peptide chain on a support instead of synthesis in solution has obvious benefits: separation of the intermediate peptides from soluble reagents and solvents can be effected simply by filtration and washing with consequent savings in time and labour over the corresponding operations in solution synthesis.

The general principles of solid phase peptide synthesis are as follows (see Fmoc Solid Phase Peptide Synthesis, A Practical Approach, by W.C. Chan and P.D. White, Oxford University Press, 2000, p. 41-76; Solid Phase Synthesis, A Practical Guide, by Steven A. Kates and Fernando Albericio, Marcel Dekker Inc., 2000, p. 275-330; Solid-Phase Peptide Synthesis, by Gregg B. Fields, Academic Press, 1997, pages 3-83): generally, the C-terminal residue of the target peptide is attached to a support. The side chains of the amino acids can also be used for attachment to the linker. Most of, or preferably all of the functional groups in amino acid side chains must be masked with permanent protecting groups that are not affected by the reaction conditions employed during peptide chain assembly. The α-amino group of each amino acid to couple, is temporarily protected. After initial attachment of the first amino acid, the temporary protecting group masking the α-amino group is removed. Thereafter, washings are performed. Then, an excess of the second amino acid is introduced, with the carboxy group of this amino acid being activated for amide bond formation by reaction with a coupling reagent. After this coupling, excess reagents are removed by washing. Then, the protecting group is removed from the N-terminus of the dipeptide. Another thorough washing takes place prior to the addition of the third amino acid residue. This process is repeated until the desired peptide sequence is assembled. In a final step, the peptide is released from the support.

Extensive washings are required after each coupling step and after each cleavage of an α-amino protecting group, i.e. after each N-deprotection step, in order to eliminate deleterious chemical entities that will otherwise lead to sequence errors in the final peptide. In particular, if an activated amino acid is still present during the N-deprotection step because the washing after the coupling was not done properly, unwanted coupling will take place with this amino acid and lead to impurities corresponding to double addition of the same amino acid in the peptide sequence. Similarly, in some cases, if the washing after the N-deprotection and before addition of a new amino acid is not done properly, cleavage reagents will remain in the solution and lead to double amino acid addition during the next coupling step.

To avoid these double addition sequences in the classical approaches, about 6 to 10 washings are necessary after each coupling and another 6 to 10 washings are necessary after each N-deprotection step. This means that for each amino acid about 12 to 20 washings are necessary in a classical approach, each of them using about 10 ml solvent / gr dry unloaded support (see Peptide Synthesis Protocols, by Michael W. Pennington and Ben M. Dunn, vol. 35, Humana Press, Chapter 1, Paragraph 3.2.1; and Fmoc Solid Phase Peptide Synthesis, A Practical Approach, by W.C. Chan and P.D. White, Oxford University Press, 2000, Table 2 on page 15 in combination with p. 43).
Reduction of the number of washing steps and the amount of solvent necessary in each individual washing step would be an important progress in terms of saving solvent and time.

This applies not only to solid phase peptide synthesis but analogously to any synthesis process wherein an oligomer is assembled on a support.
Chem Files, Green Chemistry of Fluka, vol. 1, no. 7, 2001, pages 1-18 discloses the use and advantages of quaternary ammonium salts, ionic liquids, phosphonium salts or sulfonium salts as reagents in phase transfer catalysis and other chemical reactions.
Spatola A. F. et al., "Phase transfer catalysis in solid phase peptide synthesis" Int. J. Peptide Protein Res., 40 1992, 322-332 and Chen S-T. et al. "Phase-transfer Reagents as C-Tenninal Protecting Groups" J. Chem. Soc., Chem. Commun., 1990, 1045-1047 disclose the use of phase transfer catalysis in conjunction with solid phase peptide synthesis.
Chan w.C. et al., "Fmoc solid phase peptide synthesis. A Practical Approach", 2000, Oxford University Press discloses coupling methods used in solid phase peptide synthesis.
Coste J. et al., "Coupling N-methylated amino acids using PyBroP and PyCloP halogenophosphonium salts: Mechanism and fields of application", J. Org. Chem. 1994, 59, 2437-2446 discloses the use of halogenphosphonium salts as peptide coupling reagents.
Varanda L.M. et al., "Solid-phase peptide synthesis at elevated temperatures", J. Peptide Res. 50. 1997, 102-108 discloses solid phase peptide synthesis at elevated temperatures.

The problem to be solved by the present invention was therefore to provide an improved process which greatly reduces the total amount of solvent and the process time, compared to the state of the art process.

It has now been found that addition of salts during supported phase synthesis significantly improves the wash efficiency. It is even possible to perform the process without performing any washings between the coupling and the deprotection steps.
The invention provides for a process for the solid_phase svnthesis of peptides characterised in that at least one washing step is carried out in the presence of a salt (Xⁿ⁺)ₘ(Y^{m-})ₙ, wherein X represents a cation, n represents the change of the cation, Y represents an anion and m represents the charge of the anion, and wherein said salt is selected from the group of quaternary ammonium salts, ionic liquids, phosphonium salts, inorganic cation or sulfonium salts, inorganic salts or any mixture thereof.

In a preferred aspect, the invention provides a process for attaching an α-amino protected amino acid or peptide having an unprotected C-terminus to another amino acid or peptide which is protected bv an α-amino protecting, group and which is attached to a support during solid phase peptide synthesis comprising the following steps:
a) cleave the α-amino protecting group from the amino acid or peptide attached to the support:
b) perform a thorough washing;
c) add an α-amino protected amino acid or peptide having an unprotected C-terminus and couple it to the amino acid or peptide that is attached to the support and that now has an unprotected N-terminus, to form an amide bond; and
d) perform another thorough washing;
characterized in that at least in step b) a salt (Xⁿ⁺)ₘ(Y^{m-})ₙ, which is soluble in a solvent used in this step, is added wherein X represents a cation, n represents the charge of the cation, Y represents an anion and m represents the charge of the anion, and wherein said salt is selected from the group of quaternary ammonium salts, ionic liquids, phosphonium salts, inorganic cation or sulfonium salts, inorganic salts or any mixture thereof.

Step a) is known as deprotection step and step c) is known as coupling step in solid phase peptide synthesis.

It is important that the amino acid or peptide that is added in step c) according to the invention is protected at its amino terminus but has an unprotected C-terminus.

The term peptide as used in this invention encompasses peptides of any length, including short oligopeptides such as dipeptides or tripeptides. In (Xⁿ⁺)ₘ(Y^{m-})ₙ, X represents a cation, n represents the charge of the cation, Y represents an anion and m represents the charge of the anion.

Any salt (Xⁿ⁺)ₘ(Y^{m-})ₙ that has at least 0.01% by weight solubility in the solvent may be used. Xⁿ⁺ may be any cation, but is preferably ammonium, phosphonium or sulfonium. Y^{m-} may be any anion but is preferably fluoride, chloride, bromide, iodide, hydroxide, carbonate or hydrogenocarbonate. Salts in which Y^{m-} is selected from nitrate, phosphate, hydrogenophosphate, dihydrogenophosphate, tetrafluoroborate, hexafluorophosphate, acetate, carboxylates, cyanides, isocyanates, tetra-alkylborate, tetrarylboratc, trifluoroacetate, tosylate or mesylate may be used. Preferably, the salt (Xⁿ⁺)ₘ(Y^{m-})ₙ is selected from the group of quaternary ammonium salts, ionic liquids, phosphonium salts, inorganic cation or sulfonium salts. The present invention also encompasses any mixture of these salts.

A particularly improved washing efficiency is achieved when the quaternary ammonium salt is selected from benzyltrimethylammonium hydroxide (BTMA hydroxide), benzyltrimethylammonium chloride (BTMA chloride), benzyltrimethylammonium carbonate (BTMA carbonate); the phosphonium salt is tetrabutylphosphonium bromide; and the sulfonium salt is triethylsulfonium tetrafluoroborate. Any mixture of these salts is also encompassed by this invention.

The salt (Xⁿ⁺)ₘ(Y^{m-})ₙ is typically used in a concentration of 1 to 2 weight % by volume solvent in the thorough washing. The concentration may be decreased to 0.1 weight % or increased to its solubility limit in the solvent used.

By addition of the salt (Xⁿ⁺)ₘ(Y^{m-})ₙ also in the thorough washing after cleavage of the α-amino protecting group, or in the step of coupling an α-amino protected amino acid to the peptide on the support or in the thorough washing after this coupling, the wash efficiency can be even further improved. It is therefore preferred to add the salt (Xⁿ⁺)ₘ(Y^{m-})ₙ not only in step a but additionally in any one or more of the other steps b), c) or d).

It is preferred to add the salt (Xⁿ⁺)ₘ(Y^{m-})ₙ not only in step b), but additionally in any one or more of the other steps a), c) or d).

In another preferred aspect, the invention provides a process for attaching an α-amino protected amino acid or peptide having an unprotected C-terminus to another amino acid or peptide which is protected by an α-amino protecting group and which is attached to a support during solid phase peptide synthesis comprising the following steps:
a) cleave the α-amino protecting group from the amino acid or peptide attached to the support;
b) perform a thorough washing;
c) add an α-amino protected amino acid or peptide having an unprotected C-terminus and couple it to the amino acid or peptide that is attached to the support and that now has an unprotected N-terminus, to form an amide bond; and
d) perform another thorough washing;
characterized in that at least in step c) a salt (Xⁿ⁺)ₘ(Y^{m-})ₙ, which is soluble in a solvent used in this step, is added, wherein the solvent is neither a chloroform/phenol nor a chloroform/trifluoroethanol mixture, wherein X represents a cation, n represents the charge of the cation, Y represents an anion and m represents the charge of the anion, and wherein said salt is selected from the group of quaternary ammonium salts, ionic liquids, phosphonium, salts, inorganic cation or sulfonium salts, inorganic salts or any mixture thereof.

It is preferred to add the salt (Xⁿ⁺)ₘ(Y^{m-})ₙ not only in step c), but additionally in any one or more of the other steps a), b) or d).

In another preferred aspect, the invention provides a process for attaching an α-amino protected amino acid or peptide having an unprotected C-terminus to another amino acid or peptide which is protected by an α-amino protecting group and which is attached to a support during solid phase peptide synthesis comprising the following steps:
a) cleave the α-amino protecting group from the amino acid or peptide attached to the support;
b) perform a thorough washing;
c) add an α-amino protected amino acid or peptide having an unprotected C-terminus and couple it to the amino acid or peptide that is attached to the support and that now has an unprotected N-terminus, to form an amide bond; and
d) perform another thorough washing;
characterized in that at least in step d) a salt (Xⁿ⁺)ₘ(Y^{m-})ₙ, which is soluble in a solvent used in this step, is added, wherein X represents a cation, n represents the charge of the cation, Y_represents an anion and m represents the charge of the anion and wherein said salt is selected from the groups of quaternary ammonium salts, ionic liquids, phosphonium salts, inorganic cation or sulfonium salts, inorganic salts or any mixture thereof.

It is preferred to add the salt (Xⁿ⁺)ₘ(Y^{m-})ₙ not only in step d), but additionally in any one or more of the other steps a), b) or c).

A wide variety of solvents may be chosen for all steps of the process. Preferably, the solvent is selected from DMF (N,N-dimethylformamide), NMP (N-methylpyrrolidinone), DCM (dichloromethane), ACN (acetonitrile), esters of acetic acid, acetone, dioxane, glymes ethers, low alkyl carbonates, DMSO (dimethylsulphoxide), DME (dimethoxy ethyl ether), DMEU (1,3-dimethyl-2-imidazolidinone), DMPU (1,3-dimethyl-3,4,5,6-tetrahydro-2(1H)-pyrimidinone), 1,2-dichloroethane, isopropanol, tert-butanol, methylethylketone, formamide, sulpholane, CHCl₃, CCl₄, THF (tetrahydrofuran), DMA (N,N-dimethylacetamide), supercritical fluids, ionic liquids, water or any mixture of theses solvents. Most preferably the solvent is N,N-dimethylformamide, N-methylpyrrolidinone or a mixture of them.

According to the invention, the thorough washing b) that takes place after the cleavage of the α-amino protecting group from the peptide preferably comprises not more than 8, most preferably not more than 5 washing steps. This is a considerable improvement over the prior art solid phase peptide synthesis process where about 10 washings are necessary in order to remove the cleavage reagents properly and to avoid double addition sequences.

The thorough washing d) according to the invention, which may take place after the coupling of an α-amino protected amino acid, preferably comprises not more than 5 washing steps and most preferably not more than 2 washing steps, or it can be skipped completely. This is again a considerable improvement over the prior art solid phase peptide synthesis process where 6 to 10 washings are necessary in order to properly remove excess amino acid and to avoid double addition sequences.

The volume of solvent used in each single wash step according to the invention is about 1 to 9 ml/gr dry support, compared to about 10 ml solvent / gr dry unloaded support in the prior art approach.

Dry support means the support in its dry state before any solvent has been added to it.

Generally, each washing step lasts usually for about 2 minutes and can be reduced to about 1 minute each. The individual washing time can be increased up to 60 minutes and more. The thorough washing can also be done in a continuous flow mode instead of individual washing steps, in that case, the flow rate must be adapted. The process as described above may be performed at a temperature between 0 to 50°C, preferably between 0 to 35°C and most preferably between 18 to 22°C. The process is usually performed under atmospheric pressure but it is also applicable under higher or lower pressure.

In the coupling step c) any well known coupling agent such as DIC (1,3-diisopropylcarbodiimide), DCC (1,3-dicyclohexylcarbodiimide), EDC (N-ethyl, N'- (3-dimethyl-aminopropyl)carbodiimide hydrochloride), PyBOP (Benzotriazol-1-yloxytripyrrolidinophosphonium hexafluorophosphate) or HBTU (O-benzotriazol-1-yl-N,N,N',N'-tetramethyluronium tetrafluoroborate) may be added. According to this invention, it is preferable to use DIC.

Suitable α-amino protecting groups for solid phase peptide synthesis are well known in the art (see Fmoc Solid Phase Peptide Synthesis, A Practical Approach, by W.C. Chan and P.D. White, Oxford University Press, 2000; Solid Phase Synthesis, A Practical Guide, by Steven A. Kates and Fernando Albericio, Marcel Dekker Inc., 2000; Solid-Phase Peptide Synthesis, by Gregg B. Fields, Academic Press, 1997).
One group of known α-amino protecting groups is cleavable under basic conditions. This group encompasses Fmoc (9-fluorenylmethoxycarbonyl) and Nsc (p-Nitrophenylsulphonylethoxycarbonate) but is not limited thereto. The preferred cleavage reagent for this group of α-amino protecting groups is piperidine but other bases such as morpholine may also be used. Optionally, it is possible to add DBU (1,8-diazabicyclo[5.4.0]undec-7 ene) as an additionnal cleavage reagent.

When base-cleavable α-amino protecting groups as defined above are used and the washing is not properly done, the cleavage reagents such as piperidine will remain in residual amounts in the solvent during the next coupling step. Thereby, premature cleavage of α-amino protecting groups during the coupling step takes place which again may result in double addition sequences. It has been found that addition of an acid in the thorough washing b) as defined above helps to protonate residual cleavage reagents such as piperidine, and enhance the removal efficiency of these cleavage reagents.
Any acid that is inert to the linker used on the support may be added in cycle b). It is preferably selected from HOBt (1-hydroxybenzotriazole), HOSu (N-hydroxysuccinamide), TFA (trifluoroacetic acid), HOOBt (3-hydroxy-1,2,3-benzotriazin-4(3H)-one), HOAt (7-aza-1-hydroxybenzotriazole), HCl, H₂SO₄, HNO₃, H₃PO₄. Most preferably, the acid is HOBt. If the thorough washing b) consists of 5 washing steps, it is preferred to add the acid in the second or third step.

Another group of α-amino protecting groups is cleavable under acid conditions. This group encompasses Boc (tert-butoxycarbonyl), Trt (Trityl) and Bpoc (2-p-Biphenylisopropyloxycarbonyl) but is not limited thereto. The preferred cleavage reagents, added in step a), for this group of α-amino protecting groups is TFA (trifluoroacetic acid) in DCM (dichloromethane), followed by a treatment with a base, such as TEA (triethylamine). The base is added in step b).

Another group of α-amino protecting groups considered by this invention is stable under acidic and basic conditions. Examples of such a protecting group are Benzyloxycarbonyl and Allyloxycarbonyl which may be cleaved respectively by catalytic hydrogenation or by palladium based chemistry.

All the above-mentioned embodiments concern the process of coupling a single amino acid or peptide to a another amino acid or peptide linked to a support. This process may be repeated in order to add several amino acids or peptides and to assemble a longer peptide.

Hence, this invention also relates to a process for synthesising a peptide comprising:
a') attaching a first amino acid or peptide, having an α-amino protecting group, via its C-terminus on a functionalized support;
b') perform the process as described above with the following amino acid or peptide foreseen in the sequence;
c') repeat step b') with the appropriate amino acids or peptides until the desired sequence is achieved; and
d') cleave the assembled peptide from the support by an appropriate method.

This invention also relates to the use of a salt (Xⁿ⁺)ₘ(Y^{m-})ₙ in solid phase peptide synthesis for improving the washing of the peptide resin.

More specifically, it relates to the use of a salt (Xⁿ⁺)ₘ(Y^{m-})ₙ in solid phase peptide synthesis for improving the elimination of excess amino acids or cleavage regents.

The process applicability is not scale dependent; it can for example be performed for a few milligrams or at the scale of several tons.

The following examples are provided for illustrative purposes.

### Example 1

In this comparative example, no salt (Xⁿ⁺)ₘ(Y^{m-})ₙ was used in any of the step.

A heptapeptide with the sequence Fmoc- Ser(tBu)-Tyr(tBu)-Arg(Pbf)-Lys(Boc)-Val-Leu-Gly-OH, wherein tBu (tert-butylether), Pbf (2,2,4,6,7-Pentamethyldihydrobenzofuran-5-sulfonyl) are side chain protecting groups, was synthesised on a 2-chlorotrityl polystyrene resin, according to the following protocol.
The solvent volume in liter in each wash step was 3.5 times the weight in kilogram of the starting Fmoc-Gly loaded dry resin.
1) Swell and wash 4.15 g the starting resin loaded with Fmoc-Gly-OH
2) Cleave the α-amino protecting group Fmoc by adding 3 times during 10 minutes a NMP solution comprising 1 volume % DBU and 5 volume % piperidine.
3) Wash once with NMP during 2 minutes
4) Wash once with 5 weight % HOBt in NMP during 2 minutes
5) Wash 8 times with NMP during 2 minutes
6) Add 1.98 g Fmoc-Leu-OH and couple it to the resin-linked Gly in NMP, using 1.05 ml DIC, 0.331 g HOBt. Kaiser monitoring is performed
7) Wash 9 times with NMP, during 2 minutes each
8) Treat 3 times during 10 minutes with 5 volume % piperidine / 1 volume % DBU in NMP, in order to cleave the Fmoc protecting group
9) Wash once with NMP during 2 minutes
10) Wash once with 5 weight % HOBt in NMP during 2 minutes
11) Wash 4 times with NMP during 2 minutes
12) Repeat steps 6-11 with the next amino acids Fmoc-Val-OH (1.91 g), Fmoc-Lys(Boc)-OH (2.43 g), Fmoc-Arg(Pbf)-OH (332 g), Fmoc-Tyr(tBu)-OH (2.28 g), Fmoc-Ser(tBu)-OH (1.84 g).
13) Cleave the peptide from the resin

### Example 2

In this example according to the invention, BTMA hydroxide salt was used in the washing d) after coupling.

The same heptapeptide as in Example 3 with the sequence Fmoc-Ser(tBu)-Tyr(tBu)-Arg(Pbf)-Lys(Boc)-Val-Leu-Gly-OH, wherein tBu (tert-butylether), Pbf (2,2,4,6,7-Pentamethyldihydro-benzofuran-5-sulfonyl) are side chain protecting groups, was synthesised on an identical 2-chlorotrityl polystyrene resin (same lot number) as used in Example 3, according to the following protocol.

The solvent volume in liter in each wash step was 4 times the weight in kilogram of the starting Fmoc-Gly loaded dry resin.
1) Swell and wash 4.67 g of the resin, load Fmoc-Gly-OH
2) Cleave the α-amino protecting group Fmoc by adding 3 times during 10 minutes a NMP solution comprising 1 volume % DBU and 5 volume % piperidine.
3) Wash once with NMP during 2 minutes
4) Wash once by 5 weight % HOBt in NMP during 2 minutes
5) Wash 3 times by NMP during 2 minutes
6) Add 2.23 g Fmoc-Leu-OH and couple it to the resin-linked Gly in NMP, using 1.18 ml DIC, 0.386 g HOBt. Kaiser monitoring is performed
7) Wash once with NMP, during 2 minutes and once during 10 minutes with NMP containing 2 weight % BTMA hydroxide
8) Treat 3 times during 10 minutes with 5 volume % piperidine / 1 volume % DBU in NMP, in order to cleave Fmoc
9) Wash once with NMP during 2 minutes
10) Wash once with 5 weight % HOBt in NMP during 2 minutes
11) Wash 3 times with NMP during 2 minutes
12) Repeat steps 6-11 with the next amino acids Fmoc-Val-OH (2.14 g), Fmoc-Lys(Boc)-OH (2.96 g), Fmoc-Arg(Pbf)-OH (4.09 g), Fmoc-Tyr(tBu)-OH (2.90 g), Fmoc-Ser(tBu)-OH (2.42 g).
13) Cleave the peptide from the resin

### Results

HPLC purity was analysed for most of the fragments during the synthesis of the peptide, as produced according to example 3 and 4.

| | Example 3 (without BTMA hydroxide) | Example 4 (with BTMA hydroxide) |
|---|---|---|
| Washes after coupling | 9 | 2 |
| Washes after Fmoc cleavage | 6 | 5 |
| Total amount of wash solvent per gr dry starting resin for adding one amino acid | 52.5 ml | 28 ml |
| HPLC purity of fragments: | | |
| Fmoc-Leu-Gly-OII | 94.9 % | 95.0 % |
| Fmoc-Val-Leu-Gly-OH | 90.4 % | 94.0 % |
| Fmoc-Arg(Pbf)-Lys(Boc)Val-Leu-Gly-OH | 90.0 % | 91.0 % |
| Fmoc-Ser(tBu)-Tyr(tBu)-Arg(Pbf)-Lys(Boc)Val-Leu-Gly-OH | 88.2% | 91.7% |

The HPLC results clearly show that addition of a salt as defined according to this invention in the washing after the coupling reaction gives comparable or higher purity. This result is achieved by only 7 wash steps and 28 ml solvent in total compared to 15 wash steps and 52.5 ml solvent in the comparative example.

## Claims

1. A process for the solid phase synthesis of peptides **characterised in that** at least one washing step is carried out in the presence of a salt (Xⁿ⁺)ₘ(Y^{m-})ₙ, wherein X represents a cation, n represents the charge of the cation, Y represents an anion and m represents the charge of the anion, and wherein said salt is selected from the group of quaternary ammonium salts, ionic liquids, phosphonium salts, sulfonium salts, inorganic salts or any mixture thereof.

2. The process according to claim 1 for attaching an α-amino protected amino acid or peptide having an unprotected C-terminus to another amino acid or peptide which is protected by an α-amino protecting group and which is attached to a support during solid phase peptide synthesis comprising the following steps:
a) cleave the α-amino protecting group from the amino acid or peptide attached to the support;
b) perform a thorough washing;
c) add an α-amino protected amino acid or peptide having an unprotected C-terminus and couple it to the amino acid or peptide that is attached to the support and that now has an unprotected N-terminus, to form an amide bond;
d) perform another thorough washing: and
**characterized in that** at least in step b), a salt (Xⁿ⁺)ₘ(Y^{m-})ₙ, which is soluble in a solvent used in this step, is added, wherein X represents a cation, n represents the charge of the cation, Y represents an anion and m represents the charge of the anion, and wherein said salt is selected from the group of quaternary ammonium salts, ionic liquids, phosphonium salts, sulfonium salts, inorganic salts or any mixture thereof.

3. A process for attaching an α-amino protected amino acid or peptide having an unprotected C-terminus to another amino acid or peptide which is protected by an α-amino protecting group and which is attached to a support during solid phase peptide synthesis comprising the following steps:
a) cleave the α-amino protecting group from the amino acid or peptide attached to the support;
b) perform a thorough washing;
c) add an α-amino protected amino acid or peptide having an unprotected C-terminus and couple it to the amino acid or peptide that is attached to the support and that now has an unprotected N-terminus, to form an amide bond; and
d) perform another thorough washing;
**characterized in that** at least in step c), a salt (Xⁿ⁺)ₘ(Y^{m-})ₙ, which is soluble in a solvent used in this step, is added, wherein the solvent is neither a chloroform/phenol nor a chloroform/trifluoroethanol mixture, wherein X represents a cation, n represents the charge of the cation, Y represents an anion and m represents the charge of the anion, and wherein said salt is selected from the group of quaternary ammonium salts, ionic liquids, phosphonium salts, sulfonium salts, inorganic salts or any mixture thereof.

4. The process according to claim 1 for attaching an α-amino protected amino acid or peptide having an unprotected C-terminus to another amino acid or peptide which is protected by an α-amino protecting group and which is attached to a support during solid phase peptide synthesis comprising the following steps:
a) cleave the α-amino protecting group from the amino acid or peptide attached to the support;
b) perform a thorough washing;
c) add an α-amino protected amino acid or peptide having an unprotected C-terminus and couple it to the amino acid or peptide that is attached to the support and that now has an unprotected N-terminus, to form an amide bond; and
d) perform another thorough washing;
**characterized in that** at least in step d), a salt (Xⁿ⁺)ₘ(Y^{m-})ₙ, which is soluble in a solvent used in this step, is added, wherein X represents a cation, n represents the charge of the cation, Y represents an anion and m represents the charge of the anion, and wherein said salt is selected from the group of quaternary ammonium salts, ionic liquids, phosphonium salts, sulfonium salts, inorganic salts or any mixture thereof.

5. The process according to any of claims 1 to 4, wherein (Y^{m-})ₙ is selected from the group of fluoride, chloride, bromide, iodide, hydroxide, carbonate, hydrogenocarbonate, nitrate, phosphate, hydrogenophosphate, dihydrogenophosphate, tetrafluoroborate, hexafluorophosphate, acetate, carboxylates, cyanides, isocyanates, tetra-alkylborates, tetra-arylborates, trifluoroacetate, tosylate, mesylate or any mixture thereof, wherein Y represents an anion, m represents the charge of the anion and n represents the charge of the cation.

6. The process according to any of claims 1 to 4, wherein the quaternary ammonium salt is selected from benzyltrimethylammonium hydroxide, benzyltrimethylammonium chloride or benzyltrimethylammonium carbonate, the phosphonium salt is tetrabutylphosphonium bromide and the sulfonium salt is triethylsulfonium tetrafluoroborate.

7. The process according to any of claims 2 to 6, wherein the salt added in step b), c) or d) is also added in one or more of the other steps.

8. The process according to any of claims 2 to 7, wherein the α-amino protecting group is Fmoc (9-fluorenylmethoxycarbonyl) or Nsc (p-Nitrophenylsulphonylethoxy-carbonate) or any other base-cleavable protecting group.

9. The process according to any of claims 2 to 7, wherein the α-amino protecting group is Boc (tert-butoxycarbonyl), Trt (trityl), Bpoc (2-p-Biphenylisopropyloxycarbonyl) or any other acid-cleavable protecting group.

10. The process according to any of claims 2 to 7, wherein the α-amino protecting group is selected so that neither acid nor base treatment is required for its cleavage.

11. A process for synthesising a peptide comprising:
a') attaching a first amino acid or peptide, having an α-amino protecting group, via its C-terminus to a functionalized support;
b') perform the process according to any of claims 2 to 10 with the following amino acid or peptide foreseen in the sequence;
c') repeat step b' with the appropriate amino acids or peptides until the desired sequence is achieved; and
d') cleave the assembled peptide from the support by an appropriate method.

12. Use of a salt (Xⁿ⁺)ₘ(Y^{m-})ₙ in the processes of claim 1 or 3 for improving the washing of the peptide resin, wherein X represents a cation, n represents the charge of the cation, Y represents an anion and m represents the charge of the anion.

13. Use according to claim 12 for improving the elimination of excess amino acids or cleavage reagents.

## Patentansprüche

1. Verfahren zur Festphasensynthese von Peptiden, **dadurch gekennzeichnet, dass** wenigstens ein Waschschritt in Gegenwart eines Salzes (Xⁿ⁺)ₘ(Y^{m-})ₙ durchgeführt wird, wobei X für ein Kation steht, n für die Ladung des Kations steht, Y für ein Anion steht und m für die Ladung des Anions steht, und wobei das Salz aus der aus quaternären Ammoniumsalzen, ionischen Flüssigkeiten, Phosphoniumsalzen, Sulfoniumsalzen, anorganischen Salzen und beliebigen Mischungen davon bestehenden Gruppe ausgewählt ist.

2. Verfahren nach Anspruch 1 zur Anbindung einer α-amino-geschützten Aminosäure oder eines α-amino-geschützten Peptids mit einem ungeschützten C-Terminus an eine andere Aminosäure oder ein anderes Peptid, welche bzw. welches durch eine α-Amino-Schutzgruppe geschützt ist und welche bzw. welches während der Festphasenpeptidsynthese an einen Träger gebunden ist, wobei das Verfahren die folgenden Schritte umfasst:
a) Abspaltung der α-Amino-Schutzgruppe von der an den Träger gebundenen Aminosäure bzw. dem an den Träger gebundenen Peptid;
b) Durchführen eines gründlichen Waschschritts;
c) Zugabe einer α-amino-geschützten Aminosäure oder eines α-amino-geschützten Peptids mit einem ungeschützten C-Terminus und deren bzw. dessen Kupplung an die Aminosäure bzw. das Peptid, die bzw. das an den Träger gebunden ist und nun einen ungeschützten N-Terminus aufweist, unter Bildung einer Amidbindung;
d) Durchführen eines weiteren gründlichen Waschschritts; und
**dadurch gekennzeichnet ist, dass** wenigstens in Schritt b) ein Salz (Xⁿ⁺)ₘ(Y^{m-})ₙ, welches in einem in diesem Schritt verwendeten Lösungsmittel löslich ist, zugesetzt wird, wobei X für ein Kation steht, n für die Ladung des Kations steht, Y für ein Anion steht und m für die Ladung des Anions steht, und wobei das Salz aus der aus quaternären Ammoniumsalzen, ionischen Flüssigkeiten, Phosphoniumsalzen, Sulfoniumsalzen, anorganischen Salzen und beliebigen Mischungen davon bestehenden Gruppe ausgewählt ist.

3. Verfahren zur Anbindung einer α-amino-geschützten Aminosäure oder eines α-amino-geschützten Peptids mit einem ungeschützten C-Terminus an eine andere Aminosäure oder ein anderes Peptid, welche bzw. welches durch eine α-Amino-Schutzgruppe geschützt ist und welche bzw. welches während der Festphasenpeptidsynthese an einen Träger gebunden ist, welches die folgenden Schritte umfasst:
a) Abspaltung der α-Amino-Schutzgruppe von der an den Träger gebundenen Aminosäure bzw. dem an den Träger gebundenen Peptid;
b) Durchführen eines gründlichen Waschschritts;
c) Zugabe einer α-amino-geschützten Aminosäure oder eines α-amino-geschützten Peptids mit einem ungeschützten C-Terminus und deren bzw. dessen Kupplung an die Aminosäure bzw. das Peptid, die bzw. das an den Träger gebunden ist und nun einen ungeschützten N-Terminus aufweist, unter Bildung einer Amidbindung; und
d) Durchführen eines weiteren gründlichen Waschschritts;
**dadurch gekennzeichnet ist, dass** wenigstens in Schritt c) ein Salz (Xⁿ⁺)ₘ(Y^{m-})ₙ, welches in einem in diesem Schritt verwendeten Lösungsmittel löslich ist, zugesetzt wird, wobei es sich bei dem Lösungsmittel weder um eine Chloroform/Phenol- noch eine Chloroform/Trifluorethanol-Mischung handelt, wobei X für ein Kation steht, n für die Ladung des Kations steht, Y für ein Anion steht und m für die Ladung des Anions steht, und wobei das Salz aus der aus quaternären Ammoniumsalzen, ionischen Flüssigkeiten, Phosphoniumsalzen, Sulfoniumsalzen, anorganischen Salzen und beliebigen Mischungen davon bestehenden Gruppe ausgewählt ist.

4. Verfahren nach Anspruch 1 zur Anbindung einer α-amino-geschützten Aminosäure oder eines α-amino-geschützten Peptids mit einem ungeschützten C-Terminus an eine andere Aminosäure oder ein anderes Peptid, welche bzw. welches durch eine α-Amino-Schutzgruppe geschützt ist und welche bzw. welches während der Festphasenpeptidsynthese an einen Träger gebunden ist, wobei das Verfahren die folgenden Schritte umfasst:
a) Abspaltung der α-Amino-Schutzgruppe von der an den Träger gebundenen Aminosäure bzw. dem an den Träger gebundenen Peptid;
b) Durchführen eines gründlichen Waschschritts;
c) Zugabe einer α-amino-geschützten Aminosäure oder eines α-amino-geschützten Peptids mit einem ungeschützten C-Terminus und deren bzw. dessen Kupplung an die Aminosäure bzw. das Peptid, die bzw. das an den Träger gebunden ist und nun einen ungeschützten N-Terminus aufweist, unter Bildung einer Amidbindung; und
d) Durchführen eines weiteren gründlichen Waschschritts;
**dadurch gekennzeichnet ist**, das wenigstens in Schritt d) ein Salz (Xⁿ⁺)ₘ(Y^{m-})ₙ, welches in einem in diesem Schritt verwendeten Lösungsmittel löslich ist, zugesetzt wird, wobei X für ein Kation steht, n für die Ladung des Kations steht, Y für ein Anion steht und m für die Ladung des Anions steht, und wobei das Salz aus der aus quaternären Ammoniumsalzen, ionischen Flüssigkeiten, Phosphoniumsalzen, Sulfoniumsalzen, anorganischen Salzen und beliebigen Mischungen davon bestehenden Gruppe ausgewählt ist.

5. Verfahren nach einem der Ansprüche 1 bis 4, wobei (Y^{m-})ₙ aus der aus Fluorid, Chlorid, Bromid, Iodid, Hydroxid, Carbonat, Hydrogencarbonat, Nitrat, Phosphat, Hydrogenphosphat, Dihydrogenphosphat, Tetrafluorborat, Hexafluorphosphat, Acetat, Carboxylaten, Cyaniden, Isocyanaten, Tetraalkylboraten, Tetraarylboraten, Trifluoracetat, Tosylat, Mesylat und beliebigen Mischungen davon bestehenden Gruppe ausgewählt ist, wobei Y für ein Anion steht, m für die Ladung des Anions steht und n für die Ladung des Kations steht.

6. Verfahren nach einem der Ansprüche 1 bis 4, wobei das quaternäre Ammoniumsalz aus Benzyltrimethylammoniumhydroxid, Benzyltrimethylammoniumchlorid und Benzyltrimethylammoniumcarbonat ausgewählt ist, es sich bei dem Phosphoniumsalz um Tetrabutylphosphoniumbromid handelt und es sich bei dem Sulfoniumsalz um Triethylsulfoniumtetrafluorborat handelt.

7. Verfahren nach einem der Ansprüche 2 bis 6, wobei das in Schritt b), c) oder d) zugesetzte Salz auch in einem oder mehreren der anderen Schritte zugesetzt wird.

8. Verfahren nach einem der Ansprüche 2 bis 7, wobei es sich bei der α-Amino-Schutzgruppe um Fmoc (9-Fluorenylmethoxycarbonyl) oder Nsc (p-Nitrophenylsulfonylethoxycarbonat) oder eine andere durch Basen abspaltbare Schutzgruppe handelt.

9. Verfahren nach einem der Ansprüche 2 bis 7, wobei es sich bei der α-Amino-Schutzgruppe um Boc (tert.-Butoxycarbonyl), Trt (Trityl), Bpoc (2-p-Biphenylisopropyloxycarbonyl) oder eine andere durch Säuren abspaltbare Schutzgruppe handelt.

10. Verfahren nach einem der Ansprüche 2 bis 7, wobei die α-Amino-Schutzgruppe so gewählt ist, dass zu ihrer Abspaltung weder eine Behandlung mit Säure noch eine Behandlung mit Base erforderlich ist.

11. Verfahren zur Synthese eines Peptids, bei dem man:
a') eine erste Aminosäure oder ein erstes Peptid mit einer α-Amino-Schutzgruppe über ihren bzw. seinen C-Terminus an einen funktionalisierten Träger bindet;
b') das Verfahren nach einem der Ansprüche 2 bis 10 mit der nächsten in der Sequenz vorgesehenen Aminosäure oder dem nächsten in der Sequenz vorgesehenen Peptid durchführt;
c') Schritt b' mit den entsprechenden Aminosäuren oder Peptiden wiederholt, bis man die gewünschte Sequenz erhalten hat; und
d') das zusammengefügte Peptid durch ein geeignetes Verfahren vom Träger abspaltet.

12. Verwendung eines Salzes (Xⁿ⁺)ₘ(Y^{m-})ₙ in dem Verfahren nach Anspruch 1 oder 3 zur Verbesserung des Waschens des Peptidharzes, wobei X für ein Kation steht, n für die Ladung des Kations steht, Y für ein Anion steht und m für die Ladung des Anions steht.

13. Verwendung nach Anspruch 12 zur Verbesserung der Eliminierung überschüssiger Aminosäuren oder Abspaltungsreagenzien.

## Revendications

1. Procédé pour la synthèse en phase solide de peptides, **caractérisé en ce qu'**au moins une étape de lavage est réalisée en présence d'un sel (Xⁿ⁺)ₘ(Y^{m-})ₙ, dans lequel X représente un cation, n représente la charge du cation, Y représente un anion et m représente la charge de l'anion, et dans lequel ledit sel est choisi parmi le groupe de sels d'ammonium quaternaire, de liquides ioniques, de sels de phosphonium, de sels de sulfonium, de sels inorganiques ou de tout mélange de ceux-ci.

2. Procédé selon la revendication 1 pour attacher un acide aminé ou un peptide à protection α-amino ayant une terminaison C non protégée à un autre acide aminé ou peptide qui est protégé par un groupe protecteur α-amino et qui est attaché à un support pendant la synthèse du peptide en phase solide, lequel procédé comprend les étapes suivantes :
a) clivage du groupe protecteur α-amino de l'acide aminé ou du peptide attaché au support ;
b) réalisation d'un lavage à fond ;
c) ajout d'un acide aminé ou d'un peptide à protection α-amino ayant une terminaison C non protégée et son couplage à l'acide aminé ou au peptide qui est attaché au support et qui comprend désormais une terminaison N non protégée, pour former une liaison amide ;
d) la réalisation d'un autre lavage à fond, et
**caractérisé en ce qu'**au moins à l'étape b), un sel (Xⁿ⁺)ₘ(Y^{m-})ₙ, qui est soluble dans un solvant utilisé à cette étape, est ajouté, dans lequel X représente un cation, n représente la charge du cation, Y représente un anion et m représente la charge de l'anion, et dans lequel ledit sel est choisi parmi le groupe de sels d'ammonium quaternaire, de liquides ioniques, de sels de phosphonium, de sels de sulfonium, de sels inorganiques ou de tout mélange de ceux-ci.

3. Procédé pour attacher un acide aminé ou un peptide à protection α-amino ayant une terminaison C non protégée à un autre acide aminé ou peptide qui est protégé par un groupe protecteur α-amino et qui est attaché à un support pendant la synthèse du peptide en phase solide, lequel procédé comprend les étapes suivantes :
a) le clivage du groupe protecteur α-amino de l'acide aminé ou du peptide attaché au support ;
b) la réalisation d'un lavage à fond ;
c) l'ajout d'un acide aminé ou d'un peptide à protection α-amino ayant une terminaison C non protégée et son couplage à l'acide aminé ou au peptide qui est attaché au support et qui comprend désormais une terminaison N non protégée, pour former une liaison amide ; et
d) la réalisation d'un autre lavage à fond ;
**caractérisé en ce qu'**au moins à l'étape c), un sel (Xⁿ⁺)ₘ(Y^{m-})ₙ, qui est soluble dans un solvant utilisé à cette étape, est ajouté, dans lequel le solvant n'est ni un mélange chloroforme/phénol ni un mélange chloroforme/trifluoroéthanol, dans lequel X représente un cation, n représente la charge du cation, Y représente un anion et m représente la charge de l'anion, et dans lequel ledit sel est choisi parmi le groupe de sels d'ammonium quaternaire, de liquides ioniques, de sels de phosphonium, de sels de sulfonium, de sels inorganiques ou de tout mélange de ceux-ci.

4. Procédé selon la revendication 1 pour attacher un acide aminé ou un peptide à protection α-amino ayant une terminaison C non protégée à un autre acide aminé ou peptide qui est protégé par un groupe protecteur α-amino et qui est attaché à un support pendant la synthèse du peptide en phase solide, lequel procédé comprend les étapes suivantes :
a) le clivage du groupe protecteur α-amino de l'acide aminé ou du peptide attaché au support ;
b) la réalisation d'un lavage à fond ;
c) l'ajout d'un acide aminé ou d'un peptide à protection α-amino ayant une terminaison C non protégée et son couplage à l'acide aminé ou au peptide qui est attaché au support et qui comprend désormais une terminaison N non protégée, pour former une liaison amide ; et
d) la réalisation d'un autre lavage à fond ;
**caractérisé en ce qu'**au moins à l'étape d), un sel (Xⁿ⁺)ₘ(Y^{m-})ₙ, qui est soluble dans un solvant utilisé à cette étape, est ajouté, dans lequel X représente un cation, n représente la charge du cation, Y représente un anion et m représente la charge de l'anion, et dans lequel ledit sel est choisi parmi le groupe de sels d'ammonium quaternaire, de liquides ioniques, de sels de phosphonium, de sels de sulfonium, de sels inorganiques ou de tout mélange de ceux-ci.

5. Procédé selon l'une quelconque des revendications 1 à 4, dans lequel (Y^{m-})ₙ est choisi parmi le groupe constitué du fluorure, du chlorure, du bromure, de l'iodure, de l'hydroxyde, du carbonate, de l'hydrogénocarbonate, du nitrate, du phosphate, de l'hydrogénophosphate, du dihydrogénophosphate, du tétrafluoroborate, de l'hexafluorophosphate, de l'acétate, de carboxylates, de cyanures, d'isocyanates, de tétra-alkylborates, de tétra-arylborates, du trifluoroacétate, du tosylate, du mésylate ou de tout mélange de ceux-ci, dans lequel Y représente un anion, m représente la charge de l'anion et n représente la charge du cation.

6. Procédé selon l'une quelconque des revendications 1 à 4, dans lequel le sel d'ammonium quaternaire est choisi parmi l'hydroxyde de benzyltriméthylammonium, le chlorure de benzyltriméthylammonium ou le carbonate de benzyltriméthylammonium, et dans lequel le sel de phosphonium est du bromure de tétrabutylphosphonium et le sel de sulfonium est du tétrafluoroborate de triéthylsulfonium.

7. Procédé selon l'une quelconque des revendications 2 à 6, dans lequel le sel ajouté à l'étape b), c) ou d) est également ajouté à une ou plusieurs des autres étapes.

8. Procédé selon l'une quelconque des revendications 2 à 7, dans lequel le groupe protecteur α-amino est un Fmoc (9-fluorénylméthoxycarbonyl) ou un Nsc (p-nitrophénylsulphonyléthoxy-carbonate) ou tout autre groupe protecteur clivable par une base.

9. Procédé selon l'une quelconque des revendications 2 à 7, dans lequel le groupe protecteur α-amino est un Boc (tert-butoxycarbonyl), un Trt (trityl), un Bpoc (2-p-biphénylisopropyloxycarbonyl) ou tout autre groupe protecteur clivable par un acide.

10. Procédé selon l'une quelconque des revendications 2 à 7, dans lequel le groupe protecteur α-amino est choisi de sorte qui ni un traitement acide ni un traitement basique n'est nécessaire pour son clivage.

11. Procédé pour la synthèse d'un peptide comprenant :
a') l'attachement d'un premier acide aminé ou peptide, ayant un groupe protecteur α-amino, par sa terminaison C à un support fonctionnalisé ;
b') la réalisation du procédé selon l'une quelconque des revendications 2 à 10 avec l'acide aminé ou le peptide suivant prévu dans la séquence ;
c') la répétition de l'étape b') avec les acides aminés ou peptides appropriés jusqu'à ce que la séquence souhaitée soit obtenue ; et
d') le clivage du peptide assemblé à partir du support par un procédé approprié.

12. Utilisation d'un sel (Xⁿ⁺)ₘ(Y^{m-})ₙ, dans les procédés de la revendication 1 ou 3 pour améliorer le lavage de la résine de peptide, dans lequel X représente un cation, n représente la charge du cation, Y représente un anion et m représente la charge de l'anion.

13. Utilisation selon la revendication 12 pour améliorer l'élimination de l'excès d'acides aminés ou de réactifs de clivage.
